(19) Europäisches Patentamt
European Patent Office
Office européen des brevets

(11) EP 2 143 416 B1

(12) # FASCICULE DE BREVET EUROPEEN

(45) Date de publication et mention de la délivrance du brevet:
**16.10.2013 Bulletin 2013/42**

(21) Numéro de dépôt: **09163365.1**

(22) Date de dépôt: **22.06.2009**

(51) Int Cl.:
***A61K 8/44*** (2006.01) ***A61K 8/46*** (2006.01)
***A61K 8/898*** (2006.01) ***A61Q 5/02*** (2006.01)
***A61Q 5/00*** (2006.01)

(54) **Compositions cosmétiques détergentes comprenant une silicone aminée et utilisation**

Reinigende kosmetische Zusammensetzungen enthaltend ein Aminosilikon und deren Verwendung

Detergent cosmetic compositions comprising an amino silicone, and use thereof

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **08.07.2008 FR 0854634**

(43) Date de publication de la demande:
**13.01.2010 Bulletin 2010/02**

(73) Titulaire: **L'Oréal**
**75008 Paris (FR)**

(72) Inventeurs:
- **Decoster, Sandrine**
  **95210, Saint Gratien (FR)**
- **Neplaz, Stéphanie**
  **75011, Paris (FR)**

(74) Mandataire: **Le Blainvaux Bellegarde, Françoise**
**L'Oréal**
**D.I.P.I.**
**25-29 Quai Aulagnier**
**92600 Asnières (FR)**

(56) Documents cités:
**EP-A- 1 136 066**
**EP-A- 1 312 345**
**EP-A- 1 321 131**
**EP-A- 1 543 820**
**EP-A- 1 849 453**
**WO-A-2006/065469**
**US-A1- 2003 134 760**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne de nouvelles compositions cosmétiques à propriétés améliorées destinées au nettoyage et à la protection de la couleur des fibres kératiniques telles que les cheveux, et comprenant, dans un support aqueux cosmétiquement acceptable, au moins un tensioactif anionique sulfate ou sulfonate, au moins un tensioactif amphotère et au moins une silicone aminée particulière, avec un rapport particulier en poids tensioactif amphotère / tensioactif anionique sulfate ou sulfonate. L'invention concerne aussi l'utilisation desdites compositions pour protéger la couleur artificielle des cheveux teints.

Il est connu de teindre les fibres kératiniques notamment humaines et en particulier les cheveux avec des compositions tinctoriales comprenant des précurseurs de colorant d'oxydation, appelés généralement bases d'oxydation. Ces bases d'oxydation sont des composés incolores ou faiblement colorés qui, associés à des produits oxydants, donnent naissance par un processus de condensation oxydative à des composés colorés. On sait également que l'on peut faire varier les nuances obtenues avec ces bases d'oxydation en les associant à des coupleurs ou modificateurs de coloration. La variété des molécules mises en jeu au niveau des bases d'oxydation et des coupleurs permet l'obtention d'une riche palette de couleurs.

Il est aussi connu de teindre les fibres kératiniques par une coloration directe. Le procédé classiquement utilisé en coloration directe consiste à appliquer sur les fibres kératiniques des colorants directs qui sont des molécules colorées et colorantes ayant une affinité pour les fibres, à laisser poser, puis à rincer les fibres.

Les colorations qui en résultent sont des colorations particulièrement chromatiques qui sont cependant temporaires ou semi-permanentes car la nature des interactions qui lient les colorants directs à la fibre kératinique et leur désorption de la surface et/ou du coeur de la fibre sont responsables de leur faible puissance tinctoriale et de leur mauvaise tenue aux lavages.

La couleur artificielle des cheveux apportée par un traitement de coloration directe ou d'oxydation s'estompe progressivement du fait des lavages répétés et conduit dans le temps à un affadissement de la coloration des cheveux. L'utilisation des produits soins rincés et non rincés commercialisés n'améliore pas suffisamment la tenue de la couleur artificielle des cheveux.

Il est donc nécessaire de mettre au point des moyens permettant de protéger la couleur artificielle de l'effet des lavages répétés, ceci dans des conditions douces compatibles avec les cheveux colorés.

Il existe donc un besoin d'améliorer la ténacité des colorations directes ou d'oxydation, en particulier vis à vis des shampooings.

On a déjà utilisé dans ce but des silicones aminées telles que décrite dans le document EP1312346. Cependant, les compositions divulguées ne donnent pas encore satisfaction.

Après d'importantes études sur la question, la demanderesse a découvert de manière tout à fait inattendue et surprenante que l'utilisation, d'une composition détergente comprenant une association particulière de tensioactifs et d'au moins une silicone aminée particulière, permettait de protéger la couleur artificielle de fibres kératiniques vis-à-vis des lavages répétés et ainsi d'améliorer la ténacité de la coloration.

Cette découverte est à l'origine de la présente invention.

On a en effet constaté qu'en utilisant la composition selon l'invention, on limitait l'affadissement de la coloration après plusieurs shampooings. Ainsi, la perte de coloration, évaluée par exemple à partir de la variation de la valeur du coefficient DL* des mèches avant et après shampooings dans le système CIE L*a*b* ou également à partir de DE* (correspondant à la racine carrée de la somme des carrés des variations des coefficients L*, a* et b* des mèches avant et après shampooings) était diminuée.

Les compositions conformes à l'invention confèrent également aux matières kératiniques notamment les cheveux, un remarquable effet traitant qui se manifeste notamment par une facilité de démêlage, ainsi qu'un apport de volume, de légèreté, de lissage, de douceur et de souplesse sans aucun effet de charge. Les cheveux ont un aspect naturel, propre et non gras.

Ainsi, la présente invention a pour objet de nouvelles compositions cosmétiques détergentes caractérisée par le fait qu'elles comprennent, dans un milieu aqueux cosmétiquement acceptable,

(A) un ou plusieurs tensioactifs anioniques sulfates ou sulfonates,
(B) un ou plusieurs tensioactifs amphotères et
(C) une ou un mélange de silicones aminées choisies parmi celles de formule (I) et (II),

le rapport en poids tensioactif amphotère / tensioactif anionique sulfate ou sulfonate allant de 1 à 2,
la quantité totale de tensioactifs représentant de 4 % à 35 % en poids par rapport au poids total de la composition finale.

L'invention a également pour objet l'utilisation en cosmétique des compositions ci-dessus pour le nettoyage des fibres kératiniques telles que les cheveux.

Un autre objet de l'invention concerne donc l'utilisation, en pré ou de préférence en post traitement d'une

coloration d'oxydation ou d'une coloration directe des fibres kératiniques humaines et plus particulièrement des cheveux, d'une composition détergente telle que définie ci-dessus pour protéger la couleur desdites fibres kératiniques teintes artificiellement vis-à-vis du lavage.

L'invention a également pour objet un procédé pour protéger la couleur vis-à-vis du lavage des fibres kératiniques teintes artificiellement, caractérisé par le fait qu'il consiste à appliquer sur lesdites fibres au moins une composition détergente telle que définie ci-dessus.

L'invention a également pour objet un procédé pour protéger la couleur des fibres kératiniques teintes artificiellement vis-à-vis du lavage, caractérisé par le fait qu'il consiste à appliquer sur lesdites fibres, avant, pendant ou de préférence après teinture, au moins une composition détergente telle que définie ci-dessus.

On entend par « fibres kératiniques humaines » les cheveux, les poils notamment de barbe ou moustache, les cils, les sourcils et de préférence les cheveux.

On entend par « fibres kératiniques teintes artificiellement » des fibres kératiniques teintes par un procédé de coloration directe ou par un procédé de coloration d'oxydation, de préférence par un procédé de coloration d'oxydation.

On entend par « lavage », une ou plusieurs applications sur les fibres kératiniques d'une composition aqueuse rincée, le plus souvent détergente telle qu'un shampooing. Cette expression inclut également les baignades en particulier en mer ou en piscine.

Les différents objets de l'invention vont maintenant être détaillés. L'ensemble des significations et définitions des composés utilisés dans la présente invention données ci-dessous sont valables pour l'ensemble des objets de l'invention.

(A) Tensioactifs sulfate(s) ou sulfonate(s)

Selon l'invention, les tensioactifs anioniques sulfate(s) ou sulfonate(s) sont des tensioactifs anioniques comportant au moins une fonction sulfate ($-OSO_3H$ ou $-OSO_3^-$) et/ou au moins une fonction sulfonate ($-SO_3H$ ou ($-SO_3^-$).

Les tensioactifs anioniques sulfates ou sulfonates utilisables, seuls ou mélanges, dans le cadre de la présente invention, sont les sels (en particulier sels alcalins, notamment de sodium, sels d'ammonium, sels d'amines, sels d'aminoalcools ou sels de magnésium) des alkylsulfates, alkylamidosulfates, alkyléthersulfates, alkylamidoéthersulfates, alkylaryléthersulfates, des alkyléthersulfosuccinates, des acyl iséthionates, des méthyl acyl taurates; le radical alkyle ou acyle de tous ces différents composés comportant de préférence de 8 à 24 atomes de carbone, et le radical aryle désignant de préférence un groupement phényle ou benzyle.

Le nombre moyen de groupements oxyde d'éthylène ou oxyde de propylène peut aller notamment de 2 à 50 et plus particulièrement de 2 à 10.

Parmi ces tensioactifs anioniques, on préfère utiliser les sels d'alkyléthersulfates en $C_8$-$C_{14}$ et plus particulièrement ceux en $C_{12}$-$C_{14}$. Ces sels comprennent de préférence de 2 à 5 groupements d'oxyde d'éthylène.

On utilise de préférence un agent tensioactif anionique choisi parmi les alkyl ($C_{12}$- $C_{14}$) sulfates de sodium, de triéthanolamine, de magnésium ou d'ammonium, les alkyl ($C_{12}$- $C_{14}$) éthersu! fates de sodium, d'ammonium ou de magnésium oxyéthylénés à 2, 2 moles d'oxyde d'éthylène, le cocoyl iséthionate de sodium, les méthyltaurates.

Les tensioactifs anioniques sulfates ou sulfonates sont généralement présents à raison de 2 % à 15 % en poids, de préférence de 4 à 8 % en poids, et plus particulièrement de 5 à 7% en poids par rapport au poids total de la composition.

(B) Tensioactif(s) amphotère(s) et/ou zwittérioniques:

Les agents tensioactifs amphotères et/ou zwittérioniques peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) bétaïnes ou les alkyl ($C_8$-$C_{20}$) amidoalkyl ($C_1$-$C_6$) sulfobétaïnes.

Parmi les dérivés d'amines, on peut citer les produits décrits dans les brevets US- 2 528 378 et US- 2 781 354 et de structures :

$$R_2\text{- } CONHCH_2CH_2\text{- } N\ (R_3)\ (R_4)\ (CH_2COO\text{- }) \qquad (2)$$

dans laquelle : $R_2$ CO désigne un radical acyle en C6- C24, par exemple un radical présent dans l'huile de coprah hydrolysée, un radical octoyle, décoyle ou dodécanoyle et leurs mélanges, $R_3$ désigne un groupement bêta- hydroxyéthyle et $R_4$ un groupement carboxyméthyle ;
et

$R_2$- $CONHCH_2CH_2$- N (B) (C) (3)

dans laquelle :

B représente- $CH_2CH_2OX'$, C représente- $(CH_2)_z$- Y', avec z = 1 ou 2,
X' désigne le groupement- $CH_2CH_2$- COOH ou un atome d'hydrogène
Y' désigne -COOH ou le radical -$CH_2$ - CHOH - $SO_3H$
$R'_2$ CO désigne un radical acyle en C6-C24, par exemple un radical présent dans l'huile de coprah hydrolysée ou l'huile de lin, un radical octoyle, décoyle ou dodécanoyle, stéaroyle ou isostéaroyle, oléoyle et leurs mélanges.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL® C2M concentré par la société RHODIA CHIMIE.
Selon la présente invention, on préfère plus particulièrement utiliser les agents tensio-actifs amphotères appartenant au groupe des bétaïnes tels que les alkylbétaïnes en particulier la cocobétaïne commercialisée sous la dénomination « DEHYTON AB 30 » en solution aqueuse à 30 % de MA par la société COGNIS ou les alkylamidobétaïnes, en particulier la cocamidopropylbétaine telles que la TEGOBETAINE® F50 commercialisée par la société GOLDSCHMIDT.
Le ou les tensio- actif (s) amphotère (s) et/ou zwittérioniques sont généralement présents dans des quantités allant de 2 à 30 % en poids, de préférence de 5 à 10 % en poids, par rapport au poids total de la composition.
Dans la composition selon l'invention le rapport en poids tensioactif amphotère /tensioactif anionique sulfate ou sulfonate va de 1 à 2. Plus particulièrement il va de 1,2 à 1,8, encore plus préférentiellement de 1,3 à 1,5 et encore mieux de 1,3 à 1,4.
Selon l'invention, la composition peut comprendre d'autres tensioactifs et notamment un ou plusieurs tensioactif (s) anionique(s) carboxylique(s).
Ledit (s) tensioactif (s) anionique (s) carboxylique (s) est (sont) généralement présent (s) dans des concentrations allant de 0, 5 % à 10 % en poids, de préférence de 1 à 5 % en poids par rapport au poids total de la composition.
Selon l'invention, la quantité totale de tensioactifs peut représenter de 4 % à 35 % en poids, plus particulièrement de 6 à 25% en poids, de préférence de 8 % à 20 % en poids, du poids total de la composition finale.

<u>C- Silicone aminée</u>

Les compositions de la présente invention comprennent une ou un mélange de plusieurs silicones aminées choisies parmi celles de formules (I) et (II) suivantes :

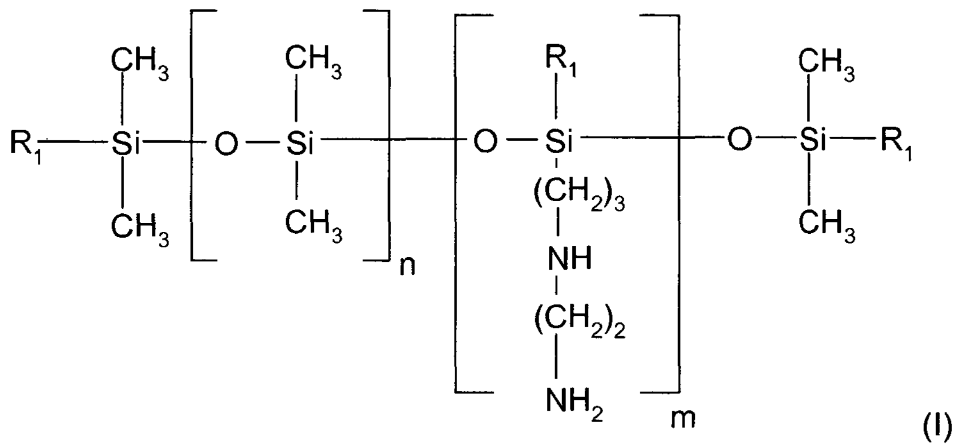

(I)

dans laquelle :

m et n sont des nombres tels que la somme (n + m) peut varier notamment de 1 à 1 000 et en particulier de 50 à

250 et plus particulièrement de 100 à 200,

n pouvant désigner un nombre de 0 à 999 et notamment de 49 à 249 et plus particulièrement de 125 à 175 et m pouvant désigner un nombre de 1 à 1 000, et notamment de 1 à 10 et plus particulièrement de 1 à 5,

$R_1$ identiques ou différents, représentent un radical hydroxy ou alcoxy en $C_1$-$C_4$, De préférence le radical alcoxy est un radical méthoxy,

une partie au moins des radicaux $R_1$ de la silicone ou du mélange de silicones de formule (I) désignant un radical alcoxy.

Le rapport molaire des radicaux Hydroxy/Alcoxy de la silicone ou du mélange de silicones de formule (I) va de préférence de 0,2 à 0,4 et plus particulièrement de 0,25 à 0,35.

La masse moléculaire moyenne en poids de la silicone va de préférence de 2000 à 1000000 et encore plus particulièrement de 3500 à 200000.

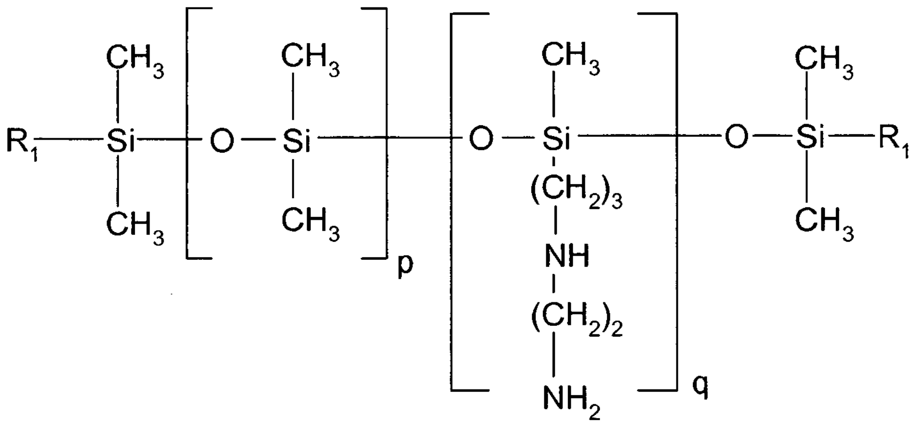

(II)

dans laquelle :

p et q sont des nombres tels que la somme (p + q) peut varier notamment de 1 à 1 000 et en particulier de 50 à 350, et plus particulièrement de 150 à 250,

p pouvant désigner un nombre de 0 à 999 et notamment de 49 à 349 et plus particulièrement de 159 à 239 et q pouvant désigner un nombre de 1 à 1 000, et notamment de 1 à 10 et plus particulièrement de 1 à 5;

$R_1$ représente un radical hydroxy ou alcoxy en $C_1$-$C_4$,

une partie au moins des radicaux $R_1$ de la silicone ou du mélange de silicones de formule (II) désignant un radical alcoxy.

Le rapport molaire des radicaux hydroxy/Alcoxy de la silicone ou du mélange de silicones de formule (II) va généralement de 0,1 à 5 et de préférence de 0,15 à 2 et encore plus particulièrement de 0,5 à 0,9. De préférence le radical alcoxy est un radical méthoxy.

La masse moléculaire moyenne en poids de la silicone va de préférence de 2000 à 200000 et encore plus particulièrement de 5000 à 100000 et plus particulièrement de 10000 à 50000.

Les masses moléculaires moyennes en poids de ces silicones aminées sont mesurées par Chromatographie par Perméation de Gel (GPC) à température ambiante en équivalent polystyrène. Les colonnes utilisées sont des colonnes μ styragel. L'éluant est le THF, le débit est de 1 ml/mn. On injecte 200 μl d'une solution à 0,5% en poids de silicone dans le THF. La détection se fait par réfractométrie et UVmétrie

Les produits commerciaux correspondant à ces silicones de structure (I) ou (II) peuvent inclure dans leur composition une ou plusieurs autres silicones aminées dont la structure est différente des formules (I) ou (II).

Un produit comprenant des silicones aminées de structure (I) est proposé par la société WACKER sous la dénomination BELSIL® ADM 652.

Un produit contenant des silicones aminées de structure (II) est proposé par WACKER sous la dénomination Fluid WR 1300® ou Fluid WR 1600®.

Lorsque ces silicones aminées sont mises en oeuvre, une forme de réalisation particulièrement intéressante est leur utilisation sous forme d'émulsion huile dans eau. L'émulsion huile dans eau peut comprendre un ou plusieurs tensioactifs.

Les tensioactifs peuvent être de toute nature mais de préférence cationique et/ou non ionique.

La taille moyenne en nombre des particules de silicone dans l'émulsion va généralement de 3 nm à 500 nanomètres .

De préférence, notamment comme silicones aminées de formule (II), on utilise des microémulsions dont la taille moyenne en volume_des particules va de 5 nm à 60 nanomètres (bornes incluses) et plus particulièrement de 5 nm à 50 nanomètres (bornes incluses).

Ainsi, on peut utiliser selon l'invention les microémulsions de silicone aminée de formule (II) proposées sous les dénominations FINISH CT 96 E®, WACKER- BELSIL ADM LOG 1 de WACKER ou SLM 28020® par la société WACKER.

De préférence la silicone aminée est choisie de façon telle que l'angle de contact avec l'eau d'un cheveu traité avec une composition contenant 2% (matière active) de ladite silicone selon l'invention varie de 90° à 180° (bornes incluses) et de préférence de 90 à 130° (bornes incluses).

Pour mesurer l'angle de contact, la silicone aminée est de préférence solubilisée ou dispersée dans un solvant de la silicone aminée ou de l'émulsion de silicone aminée (notamment l'hexaméthyldisiloxane ou l'eau selon l'hydrophilie de la silicone).

De préférence la composition contenant la ou les silicones aminées de formule (I) ou (II) est telle que l'angle de contact avec l'eau d'un cheveu traité avec ladite composition varie de 90 à 180°(bornes incluses) et de préférence de 90 à 130° (bornes incluses).

La mesure de l'angle de contact est basée sur l'immersion d'un cheveu dans de l'eau distillée. Il consiste à évaluer la force exercée par l'eau sur le cheveu lors de son immersion de l'eau distillée et lors de son retrait. Les forces ainsi mesurées sont directement reliées à l'angle de contact $\theta$ entre l'eau et la surface du cheveu. Le cheveu est dit hydrophile lorsque l'angle $\theta$ est situé de 0 à 90° (bornes incluses), hydrophobe lorsque cet angle est situé de 90° à 180° (bornes incluses).

Le test s'effectue avec des mèches de cheveux naturels ayant été décolorés dans les mêmes conditions puis lavés.

Chaque mèche de 1 g est placée dans un cristallisoir de 75 mm de diamètre puis recouverte de façon homogène par 5 mL de la formule à tester. La mèche est ainsi laissée 15 minutes à température ambiante puis rincée à l'eau distillée pendant 30 secondes. La mèche essorée est laissée à l'air libre jusqu'à ce qu'elle soit complètement sèche.

Pour chaque évaluation, 10 cheveux ayant subi le même traitement sont analysés. Chaque échantillon, fixé à une microbalance de précision, est immergé par la pointe dans un récipient rempli d'eau distillée. Cette balance DCA (« Dynamic Contact Angle Analyser »), de la société CAHN Instruments, permet de mesurer la force (F) exercée par l'eau sur le cheveu.

Parallèlement, le périmètre du cheveu (P) est mesuré *via* une observation microscopique.

La force moyenne de mouillabilité sur 10 cheveux et la section des cheveux analysés permettent d'obtenir l'angle de contact du cheveu sur l'eau, selon la formule :

$$F = P * \Gamma lv * \cos\theta$$

où F est la force de mouillabilité exprimée en Newton, P le périmètre du cheveu en mètre, $\Gamma lv$ la tension interfaciale liquide / vapeur de l'eau en $J/m^2$ et $\theta$ l'angle de contact.

Le produit SLM 28020 de WACKER à 12% dans l'eau (soit 2% en silicone aminée) conduit à un angle de contact de 93° selon le test indiqué ci-dessus.

Le produit BELSIL ADM 652 de WACKER à 2% dans l'hexaméthyldisiloxane (soit 2% en silicone aminée) conduit à un angle de contact de 111° selon le test indiqué ci-dessus.

Dans un mode avantageux de l'invention , les compositions de l'invention contiennent une ou plusieurs silicones aminées de formule (II).

La ou les silicones aminées de formules (I) ou (II) sont utilisées de préférence en une quantité allant de 0,01 à 20% en poids par rapport au poids total de la composition. Plus préférentiellement, cette quantité va de 0,05 à 10% en poids et encore plus particulièrement de 0,1 à 5 % en poids par rapport au poids total de la composition.

Selon un mode de réalisation de l'invention, les compositions peuvent comprendre en outre un sel hydrosoluble et/ou un alcool hydrosoluble mono ou polyhydroxylé. Les sels hydrosolubles selon l'invention sont de préférence les sels de métaux mono ou divalents et d'un acide minéral ou organique.

On peut citer en particulier le chlorure de sodium, le chlorure de potassium, le chlorure de calcium, le sulfate de magnésium, le citrate de sodium, les sels de sodium de l'acide phosphorique. Le chlorure de sodium est particulièrement préféré.

Comme alcool hydrosoluble mono ou polyhydroxylé on peut citer l'éthanol, l'isopropanol, le propylèneglycol,

le glycérol, l'hexylèneglycol.

Les compositions détergentes selon l'invention présentent un pH final généralement compris entre 2 et 8. De préférence, ce pH est compris entre 3 et 7, 5. L'ajustement du pH à la valeur désirée peut se faire classiquement par ajout d'une base (organique ou minérale) dans la composition, par exemple de la soude, de l'ammoniaque ou une (poly) amine primaire, secondaire ou tertiaire comme la monoéthanolamine, la diéthanolamine, la triéthanolamine, l'isopropanolamine ou la propanediamine- 1, 3, ou encore par ajout d'un acide minéral ou organique, de préférence l'acide citrique ou l'acide chlorhydrique.

Le milieu aqueux cosmétiquement acceptable peut être constitué uniquement par de l'eau ou par un mélange d'eau et d'un solvant cosmétiquement acceptable tel que les alcools mono ou polyhydroxylés mentionnés ci-dessus ou les éthers de ces alcools, les esters, les cétones.

La composition selon l'invention comprend de préférence au moins 30% en poids d'eau et plus particulièrement de 50 à 90% en poids et encore préférentiellement de 70 à 85% en poids par rapport au poids total de la composition.

La composition comprend de préférence moins de 20% en poids de phase grasse, par rapport au poids total de la composition.

La phase grasse comprend tous les corps gras insolubles dans l'eau à température ambiante de la composition tels que notamment les esters gras, les huiles végétales, minérales, synthétiques, les alcools gras, les acides gras, les amides gras, les cires, les silicones. La phase grasse représente de préférence de 0,1 à 15% en poids et plus particulièrement de 0,5 à 10% en poids par rapport au poids total de la composition et encore plus particulièrement de 0,5 à 8% en poids.

Les compositions conformes à l'invention peuvent contenir en plus de l'association définie ci-dessus des agents régulateurs de viscosité tels que des agents épaississants. On peut citer en particulier les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en $C_{10}$-$C_{30}$ réticulés. Ces agents régulateurs de viscosité sont utilisés dans les compositions selon l'invention dans des proportions pouvant aller jusqu'à 10 % en poids par rapport au poids total de la composition. On utilisera de préférence les acides polyacryliques réticulés tels que par exemple le Carbopol 980 de NOVEON

Les compositions conformes à l'invention peuvent également contenir jusqu'à 5 % d'agents nacrants ou opacifiants bien connus dans l'état de la technique tels que par exemple les alcools gras, les palmitates de sodium ou de magnésium, les stéarates et hydroxystéarates de sodium ou de magnésium, les alcool gras, les dérivés acylés à chaîne grasse tels que les distéarates d'éthylène glycol ou de polyéthylèneglycol, les éthers à chaînes grasses tels que par exemple le distéaryléther ou le 1- (hexadécyloxy)- 2- octadécanol, les cyclodextrines en particulier les β- cyclodextrines.

Les compositions conformes à l'invention peuvent éventuellement contenir en outre d'autres agents ayant pour effet d'améliorer les propriétés cosmétiques de cheveux ou de la peau sans cependant altérer la stabilité des compositions. On peut citer à ce sujet les agents tensioactifs cationiques, les polymères anioniques ou non ioniques ou cationiques ou amphotères, les protéines, les hydrolysats de protéines, les céramides, les pseudocéramides, les huiles végétales, les acides gras notamment à chaînes linéaires ou ramifiées en $C_{16}$-$C_{40}$ tels que l'acide méthyl-18 eicosanoïque, les hydroxyacides, les vitamines, les provitamines telle que le panthénol, les silicones, volatiles ou non volatiles, solubles et insolubles dans le milieu différentes des silicones aminées de formules (I) ou (II), les filtres UV, les agents hydratants, les agents antipelliculaires ou antiséborrhéiques, les agents antichute des cheveux, les agents anti-radicaux libres, et leurs mélanges.

Selon un mode particulièrement préféré, les compositions selon l'invention comprennent en outre un ou plusieurs polymères cationiques non siliconés.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression « polymère cationique » désigne tout polymère contenant des groupements cationiques et/ou des groupements ionisables en groupements cationiques.

Les polymères cationiques utilisables selon l'invention ont de préférence une densité de charge cationique supérieure ou égale à 0,01 meq./g, et plus particulièrement allant de 0,1 à 10 meq./g et en particulier de 3 à 8 meq/g.

Parmi tous les polymères cationiques susceptibles d'être utilisés dans le cadre de la présente invention, on préfère mettre en oeuvre les polysaccharides cationiques et notamment les dérivés d'éther de cellulose quaternaires tels que les produits commercialisés sous la dénomination «UCARE POLYMER JR 400 » par la société AMERCHOL, les cyclopolymères, en particulier les homopolymères de sel de diallyldiméthylammonium en particulier le MERQUAT 100 et les copolymères de sel de diallyldiméthylammonium et d'acrylamide en particulier les chlorures, commercialisés sous les dénominations « MERQUAT 550 » et « MERQUAT S » par la société NALCO, les gommes de guar modifiées

par du chlorure de 2, 3- époxypropyl triméthylammonium commercialisées par exemple sous la dénomination « JAGUAR C13S » par la société RHODIA CHIMIE, les homopolymères et les copolymères éventuellement réticulés de sel de (méth) acryloyloxyéthyltriméthylammonium, vendus par exemple par la société CIBA en solution à 50% dans de l'huile minérale sous les dénominations commerciales SALCARE SC92 (copolymère réticulé du chlorure de méthacryloyloxyéthyltriméthylammonium et de l'acrylamide) et SALCARE SC95 (homopolymère réticulé du chlorure de méthacryloyloxyéthyl triméthylammonium), les copolymères quaternaires de vinylpyrrolidone et de sel de vinyl imidazole tels que les produits commercialisés par BASF sous les dénominations LUVIQUAT FC 370, LUVIQUAT FC 550, LUVIQUAT FC 905 et LUVIQUAT HM- 552.

Les polysaccharides cationiques, les homopolymères et les copolymères de sels (chlorure) de diallyldiméthylammomnium sont particulièrement préférés.

Selon l'invention, le ou les polymères cationiques peuvent représenter de 0,005 % à 10 % en poids, de préférence de 0,01 % à 5 % en poids, et encore plus préférentiellement de 0,1 % à 3 % en poids, du poids total de la composition finale.

Les compositions selon l'invention peuvent contenir également des synergistes de mousses tels que des 1,2-alcanediols en $C_{10}$-$C_{18}$ ou des alcanolamides gras en $C_{10}$-$C_{18}$ dérivés de mono ou de diéthanolamine.

Bien entendu, l'homme du métier veillera à choisir ce ou ces éventuels composés complémentaires et/ou leurs quantités de manière telle que la stabilité des compositions et les propriétés cosmétiques attachées intrinsèquement à la composition conforme à l'invention ne soient pas, ou substantiellement pas, altérées par la ou les adjonctions envisagées.

Selon un mode particulier de l'invention, les compositions selon l'invention sont transparentes.

La transparence peut se mesurer par mesure de la transmittance à 700nm via un spectromètre (par exemple spectromètre Lambda 14 de Perkin Elmer ou UV2101PC de Shimadzu). Les compositions transparentes ont une transmittance supérieure ou égale à 94%, de préférence de 96 à 100%.

Ces compositions peuvent se présenter sous la forme de liquides plus ou moins épaissis, de crèmes ou de gels et elles conviennent principalement au lavage, au soin des fibres kératiniques en particulier des cheveux.

L'invention a également pour objet un procédé de lavage et de conditionnement des fibres kératiniques telles que notamment les cheveux consistant à appliquer sur lesdites matières mouillées une quantité efficace d'une composition telle que définie ci-dessus, puis à effectuer un rinçage à l'eau après un éventuel temps de pause.

Les compositions selon l'invention sont utilisées de préférence comme shampooings pour le lavage et le conditionnement des cheveux et ils sont appliqués dans ce cas-là sur les cheveux humides dans des quantités efficaces pour les laver, et la mousse générée par massage ou friction avec les mains est ensuite éliminée après un éventuel temps de pause, par rinçage avec de l'eau, l'opération pouvant être répétée une ou plusieurs fois.

Des exemples concrets, mais nullement limitatifs, illustrant l'invention vont maintenant être donnés.

## EXEMPLE 1

On a réalisé la composition de shampooing conforme à l'invention

| | 1 |
|---|---|
| Lauryléthersulfate de sodium (C12/C14 à 70/30) à 2,2 moles d'oxyde d'éthylène en solution aqueuse à 70% de MA | 5,6 gMA |
| Cocobétaïne à 30% MA (DEHYTON AB 30 de COGNIS) | 7,5 gMA |
| Acide lauryléther carboxylique à 90% MA dans l'eau (AKYPO RLM 45 CA de KAO) | 2.7 gMA |
| Silicone aminée (WACKER-BELSIL ADM LOG 1 de WACKER SILICONES) | 1,35 g MA |
| Chlorure de poly-diméthyl diallyl ammonium dans l'eau à 40 % de matière active (MERQUAT 100 de NALCO) | 0,24 gMA |
| Chlorure de sodium | 1 g |
| Alcool cétylstéarylique oxyéthyléné à 60 OE éther de myristylglycol-1,2 (ELFACOS GT 282 S de AKZO NOBEL) | 3,3 g |
| - Parfum, conservateur | qs |
| - Acide chlorhydrique qs pH | 5,3 |
| - Eau déminéralisée qsp | 100 g |

Les cheveux traités avec ces compositions se démêlent facilement et sont légers et lisses de la racine à la pointe.
Les cheveux teints artificiellement traités avec cette composition présentent une bonne ténacité de la couleur aux shampooings répétés.

**EXEMPLE 2**

On a réalisé les compositions de shampooing suivantes :

| | 2 (invention) | A |
|---|---|---|
| Lauryléthersulfate de sodium (C12/C14 à 70/30) à 2,2 moles d'oxyde d'éthylène en solution aqueuse à 70% de MA | 5,56 gMA | 7 gMA |
| Cocobétaïne à 30% MA (DEHYTON AB 30 de COGNIS) | 7,5 gMA | 2,5 gMA |
| Silicone aminée (WACKER-BELSIL ADM LOG 1 de WACKER SILICONES) | 1,5 g MA | 1,5 g MA |
| Hydroxyéthylcellulose quaternisée par du 2,3-époxypropyl triméthylammonium (UCARE POLYMER JR 400 de AMERCHOL) | 0,4 gMA | 0,4 gMA |
| Distéarate d'éthylèneglycol | 1,5 g | 1,5 g |
| Polymère acrylique en émulsion (AQUA SF1 de NOVEON) | 0,8 gMA | 0,8 gMA |
| - Conservateur | qs | qs |
| - Acide citrique ou NaOH qs pH | 5,5 | 5,5 |
| - Eau déminéralisée qsp | 100 g | 100 g |
| | | |
| Rapport en poids tensioactif amphotère / tensioactif anionique sulfate | 1,35 | 0,36 |

Après 8 shampooings (0,4 g de shampooing par g de cheveux), on a obtenu sur des cheveux naturels 90% blancs permanentés teints par une coloration Majirouge 6.66 de l'OREAL (4g de crème et 6 g d'oxydant 20 volumes) un DE (écart de couleur avant et après traitement par les 8 shampooings) de 8,75 contre un DE de 13,3 obtenu pour les cheveux traités avec le shampooing A.
Plus le DE est petit, plus la protection est efficace.
Les cheveux teints artificiellement traités avec la composition 2 ont présenté une meilleure ténacité de la couleur aux shampooings répétés que ceux traités avec la composition A.

**Revendications**

1. Composition cosmétique détergente, **caractérisée par le fait qu'**elle comprend, dans un milieu aqueux cosmétiquement acceptable,

    (A) un ou plusieurs tensioactifs anioniques sulfate ou sulfonate,
    (B) un ou plusieurs tensioactifs amphotères et
    (C) une ou un mélange de silicones aminées de formule (I) ou (II) suivante,

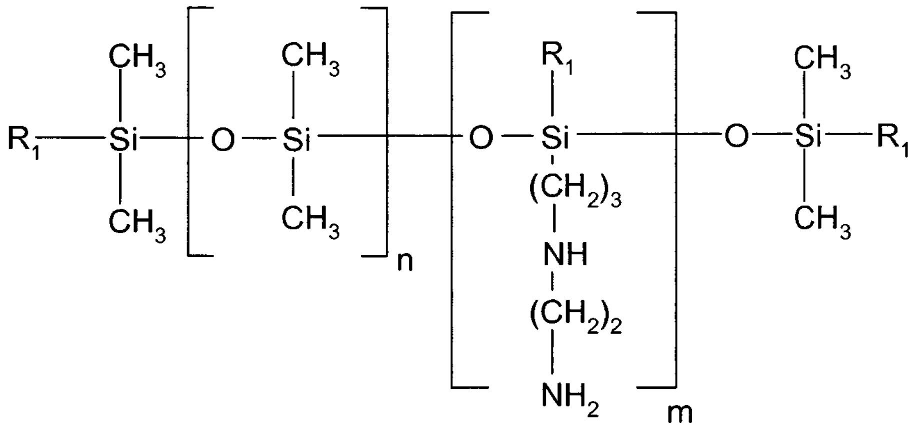

(I)

dans laquelle :

m et n sont des nombres tels que la somme (n + m) peut varier notamment de 1 à 1 000 et en particulier de 50 à 250 et plus particulièrement de 100 à 200,
n pouvant désigner un nombre de 0 à 999 et notamment de 49 à 249 et plus particulièrement de 125 à 175 et m pouvant désigner un nombre de 1 à 1 000, et notamment de 1 à 10 et plus particulièrement de 1 à 5,
$R_1$ identiques ou différents, représentent un radical hydroxy ou alcoxy en $C_1$-$C_4$,

De préférence le radical alcoxy est un radical méthoxy,
une partie au moins des radicaux $R_1$ de la silicone ou du mélange de silicones de formule (I) désignant un radical alcoxy.

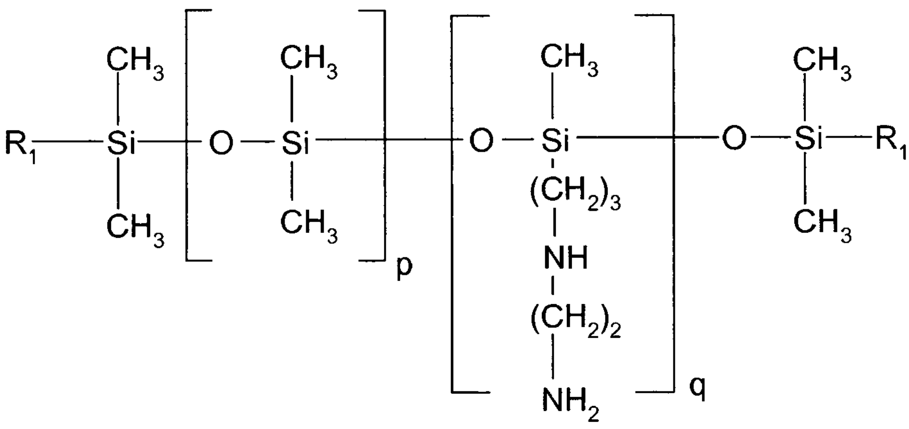

(II)

dans laquelle :

p et q sont des nombres tels que la somme (p + q) peut varier notamment de 1 à 1 000 et en particulier de 50 à 350, et plus particulièrement de 150 à 250,
p pouvant désigner un nombre de 0 à 999 et notamment de 49 à 349 et plus particulièrement de 159 à 239 et q pouvant désigner un nombre de 1 à 1 000, et notamment de 1 à 10 et plus particulièrement de 1 à 5;
$R_1$ représente un radical hydroxy ou alcoxy en $C_1$-$C_4$,
une partie au moins des radicaux $R_1$ de la silicone ou du mélange de silicones de formule (II) désignant un radical alcoxy,

le rapport en poids tensioactif amphotère / tensioactif anionique sulfate ou sulfonate allant de 1 à 2,
la quantité totale de tensioactifs représentant de 4 % à 35 % en poids par rapport au poids total de la composition finale.

2. Composition selon la revendication 1, **caractérisée en ce que** le(s) tensioactif(s) anionique(s) sulfate ou sulfonate est (sont) présent(s) dans des concentrations allant de 2 à 15 % en poids, de préférence de 4 à 8 % en poids, par rapport au poids total de la composition.

3. Composition selon l'une quelconque des revendications 1 à 2, **caractérisée en ce que** le(s) tensioactif(s) amphotère (s) est(sont) présent(s) dans des concentrations allant de 2 à 30 % en poids, de préférence de 5 à 10 % en poids, par rapport au poids total de la composition.

4. Composition selon l'une quelconque des revendications 1 à 3, **caractérisée par le fait que** le rapport en poids tensioactif amphotère /tensioactif anionique sulfate ou sulfonate va de 1,2 à 1,8, plus particulièrement de 1,3 à 1,5.

5. Composition selon l'une quelconque des revendications 1 à 4, **caractérisée par le fait que** dans la silicone aminée de formule (I) ou (II), les radicaux alcoxy sont des radicaux méthoxy,

6. Composition selon l'une quelconque des revendications 1 à 5, **caractérisée par le fait que** dans la silicone ou le mélange de silicones aminées de formule (I) le rapport molaire des radicaux Hydroxy/Alcoxy de la silicone ou du mélange de silicones de formule (I) va de 0,2 à 0,4 et de préférence de 0,25 à 0,35 .

7. Composition selon l'une quelconque des revendications 1 à 5, **caractérisée par le fait que** dans la silicone ou le mélange de silicones aminées de formule (II) le rapport molaire des radicaux Hydroxy/Alcoxy de la silicone ou du mélange de silicones de formule (II) va de 0,1 à 0,5, de préférence de 0,15 à 2 et encore plus particulièrement de 0,5 à 0,9.

8. Composition selon l'une quelconque des revendications précédentes, **caractérisée par le fait que** les silicones aminées de formules (I) ou (II) sont utilisées sous forme de microémulsion.

9. Composition selon l'une quelconque des revendications précédentes, **caractérisée par le fait que** les silicones aminées de formules (I) ou (II) sont présentes dans des concentrations allant de 0,01 à 20 %, et de préférence de 0,05 à 10 % en poids et encore plus particulièrement de 0,1 à 5% en poids par rapport au poids total de la composition.

10. Composition selon l'une quelconque des revendications précédente, **caractérisée en ce qu'**elle comprend en outre un ou plusieurs tensioactif(s) anionique(s) carboxylique(s).

11. Composition selon l'une quelconque des revendications précédentes, **caractérisée par le fait que** la composition comprend en outre un ou plusieurs polymères cationiques non siliconés.

12. Composition selon la revendication 11, **caractérisée par** le fait le polymère cationique est choisi parmi les homopolymères de sel de diallyldiméthylammonium et les polysaccharides cationiques.

13. Composition selon l'une quelconque des revendications 11 à 12, **caractérisée en ce que** ledit polymère cationique représente de 0,005 % à 10 % en poids, de préférence de 0,01 % à 5 % en poids, et encore plus préférentiellement de 0,1 % à 3 % en poids, du poids total de la composition finale.

14. Procédé pour protéger la couleur vis-à-vis du lavage des fibres kératiniques teintes artificiellement notamment des fibres kératiniques humaines et plus particulièrement des cheveux, **caractérisé par le fait qu'**il consiste à appliquer sur lesdites fibres, avant ou de préférence après teinture desdites fibres, au moins une composition telle définie à l'une des revendications 1 à 13.

15. Utilisation d'une composition telle que définie à l'une des revendications 1 à 13 pour protéger la couleur vis-à-vis du lavage des fibres kératiniques teintes artificiellement notamment des fibres kératiniques humaines et plus particulièrement des cheveux.

**Patentansprüche**

1. Kosmetische Reinigungszusammensetzung, **dadurch gekennzeichnet, dass** sie in einem kosmetisch unbedenklichen wässrigen Medium:

(A) ein oder mehrere anionische Sulfat- oder Sulfonat-Tenside,
(B) ein oder mehrere amphotere Tenside und
(C) ein Aminosilikon oder ein Gemisch von Aminosilikonen der folgenden Formel (I) oder (II),

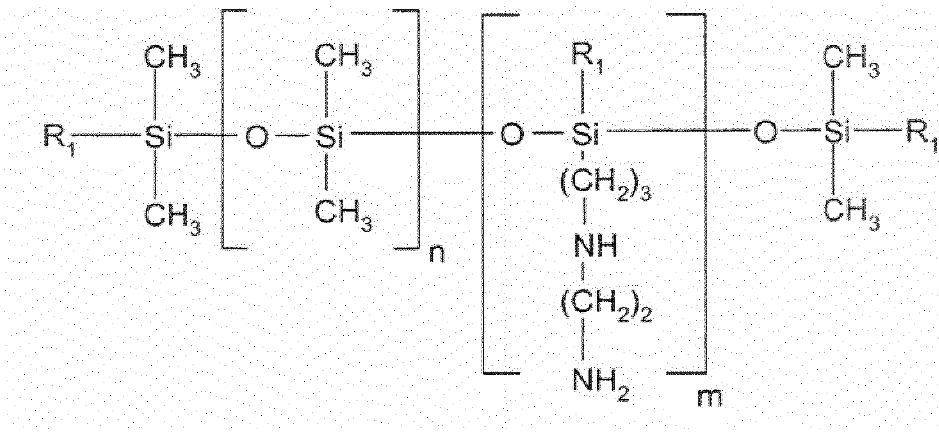

(I)

worin:

m und n solche Zahlen sind, dass die Summe (n + m) insbesondere von 1 bis 1000 und speziell von 50 bis 250 und spezieller von 100 bis 200 variieren kann,
n für eine Zahl von 0 bis 999 und insbesondere von 49 bis 249 und spezieller von 125 bis 175 stehen kann und m für eine Zahl von 1 bis 1000 und insbesondere von 1 bis 10 und spezieller von 1 bis 5 stehen kann,

die Reste $R_1$ gleich oder verschieden sind und für einen Hydroxy- oder $C_1$- $C_4$- Alkoxyrest stehen, wobei es sich bei dem Alkoxyrest vorzugsweise um einen Methoxyrest handelt,
wobei mindestens ein Teil der Reste $R_1$ des Silikons bzw. des Gemischs von Silikonen der Formel (I) für einen Alkoxyrest steht;

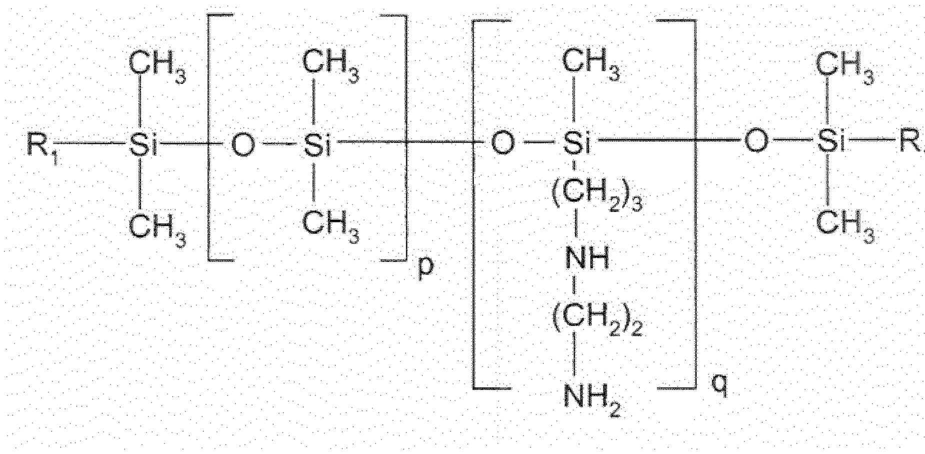

(II)

worin:

p und q solche Zahlen sind, dass die Summe (p + q) insbesondere von 1 bis 1000 und speziell von 50 bis 350 und spezieller von 150 bis 250 variieren kann,
p für eine Zahl von 0 bis 999 und insbesondere von 49 bis 349 und spezieller von 159 bis 239 stehen kann und 9 für eine Zahl von 1 bis 1000 und insbesondere von 1 bis 10 und spezieller von 1 bis 5 stehen kann,
$R_1$ für einen Hydroxy- oder $C_1$- $C_4$- Alkoxyrest steht, wobei mindestens ein Teil der Reste $R_7$ des Silikons bzw. des Gemischs von Silikonen der Formel (II) für einen Alkoxyrest steht,
wobei das Gewichtsverhältnis von amphoterem Tensid zu anionischem Sulfat- oder Sulfonat-Tensid im Bereich von 1 bis 2 liegt,
wobei die Gesamtmenge an Tensid 4 bis 35 Gew. -%, bezogen auf das Gesamtgewicht der fertigen Zusammensetzung, ausmacht, umfasst.

2. Zusammensetzung nach Anspruch 1, **dadurch gekennzeichnet, dass** das anionische Sulfat- oder Sulfonat-Tensid bzw. die anionischen Sulfat- oder Sulfonat-Tenside in Konzentrationen im Bereich von 2 bis 15 Gew.-%, vorzugsweise 4 bis 8 Gew.-%, bezogen auf das Gesamtgewicht der Zusammensetzung, vorliegt bzw. vorliegen.

3. Zusammensetzung nach einem der Ansprüche 1 bis 2, **dadurch gekennzeichnet, dass** das amphotere Tensid bzw. die amphoteren Tenside in Konzentrationen im Bereich von 2 bis 30 Gew.-%, vorzugsweise 5 bis 10 Gew.-%, bezogen auf das Gesamtgewicht der Zusammensetzung, vorliegt bzw. vorliegen.

4. Zusammensetzung nach einem der Ansprüche 1 bis 3, **dadurch gekennzeichnet, dass** das Gewichtsverhältnis von amphoterem Tensid zu anionischem Sulfat- oder Sulfonat-Tensid im Bereich von 1,2 bis 1,8 und spezieller 1,3 bis 1,5 liegt.

5. Zusammensetzung nach einem der Ansprüche 1 bis 4, **dadurch gekennzeichnet, dass** es sich in dem Aminosilikon der Formel (I) oder (II) bei den Alkoxyresten um Methoxyreste handelt.

6. Zusammensetzung nach einem der Ansprüche 1 bis 5, **dadurch gekennzeichnet, dass** in dem Aminosilikon bzw. dem Gemisch von Aminosilikonen der Formel (I) das Molverhältnis von Hydroxyresten zu Alkoxyresten des Silikons bzw. des Gemischs von Silikonen der Formel (I) im Bereich von 0,2 bis 0,4 und vorzugsweise 0,25 bis 0,35 liegt.

7. Zusammensetzung nach einem der Ansprüche 1 bis 5, **dadurch gekennzeichnet, dass** in dem Aminosilikon bzw. dem Gemisch von Aminosilikonen der Formel (II) das Molverhältnis von Hydroxyresten zu Alkoxyresten des Silikons bzw. des Gemischs von Silikonen der Formel (II) im Bereich von 0,1 bis 0,5, vorzugsweise 0,15 bis 2 und noch spezieller 0,5 bis 0,9 liegt.

8. Zusammensetzung nach einem der vorhergehenden Ansprüche, **dadurch gekennzeichnet, dass** die Aminosilikone der Formeln (I) oder (II) in Form einer Mikroemulsion verwendet werden.

9. Zusammensetzung nach einem der vorhergehenden Ansprüche, **dadurch gekennzeichnet, dass** die Aminosilikone der Formeln (I) oder (II) in Konzentrationen im Bereich von 0,01 bis 20 Gew.-% und vorzugsweise 0,05 bis 10 Gew.-% und noch spezieller 0,1 bis 5 Gew.-%, bezogen auf das Gesamtgewicht der Zusammensetzung, vorliegen.

10. Zusammensetzung nach einem der vorhergehenden Ansprüche, **dadurch gekennzeichnet, dass** sie außerdem ein oder mehrere carboxylgruppenhaltige anionische Tenside umfasst.

11. Zusammensetzung nach einem der vorhergehenden Ansprüche, **dadurch gekennzeichnet, dass** die Zusammensetzung außerdem ein oder mehrere kationische Nichtsilikonpolymere umfasst.

12. Zusammensetzung nach Anspruch 11, **dadurch gekennzeichnet, dass** das kationische Polymer aus Diallyldimethylammoniumsalz-Homopolymeren und kationischen Polysacchariden ausgewählt ist.

13. Zusammensetzung nach einem der Ansprüche 11 bis 12, **dadurch gekennzeichnet, dass** das kationische Polymer 0,005 bis 10 Gew.-%, vorzugsweise 0,01 bis 5 Gew.-% und noch weiter bevorzugt 0,1 bis 3 Gew.-% des Gesamtgewichts der fertigen Zusammensetzung ausmacht.

14. Verfahren zum Schützen der Farbe künstlich gefärbter Keratinfasern, insbesondere menschlicher Keratinfasern und spezieller der Haare, gegenüber Waschen, **dadurch gekennzeichnet, dass** es daraus besteht, dass man auf die Fasern vor oder vorzugsweise nach dem Färben der Fasern mindestens eine Zusammensetzung gemäß einem der Ansprüche 1 bis 13 aufbringt.

15. Verwendung einer Zusammensetzung gemäß einem der Ansprüche 1 bis 13 zum Schützen der Farbe künstlich gefärbter Keratinfasern, insbesondere menschlicher Keratinfasern und spezieller der Haare, gegenüber Waschen.

## Claims

1. Detergent cosmetic composition, **characterized in that** it comprises, in a cosmetically acceptable aqueous medium:

(A) one or more sulfate or sulfonate anionic surfactants,
(B) one or more amphoteric surfactants and
(C) one or a mixture of amino silicones of formula (I) or (II) below:

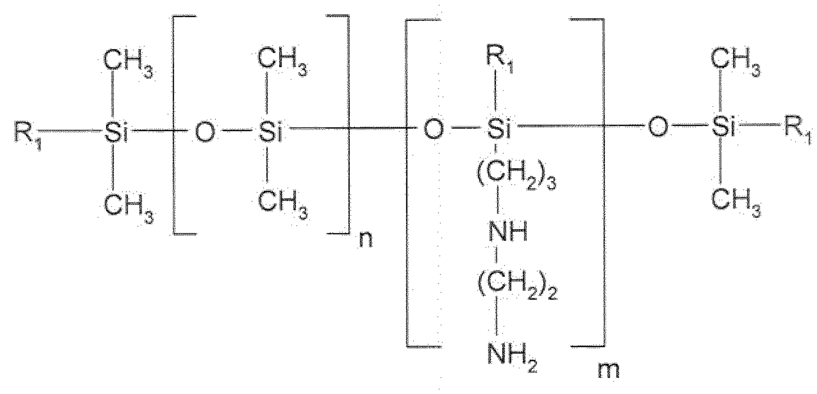

(I)

in which:

m and n are numbers such that the sum (n + m) can range especially from 1 to 1000, in particular from 50 to 250 and more particularly from 100 to 200,
n possibly denoting a number from 0 to 999, especially from 49 to 249 and more particularly from 125 to 175 and m possibly denoting a number from 1 to 1000, especially from 1 to 10 and more particularly from 1 to 5,
$R_1$, which may be identical or different, represent a hydroxyl or $C_1$-$C_4$ alkoxy radical,

preferably, the alkoxy radical is a methoxy radical,
at least some of the radicals $R_1$ of the silicone or of the mixture of silicones of formula (I) denoting an alkoxy radical;

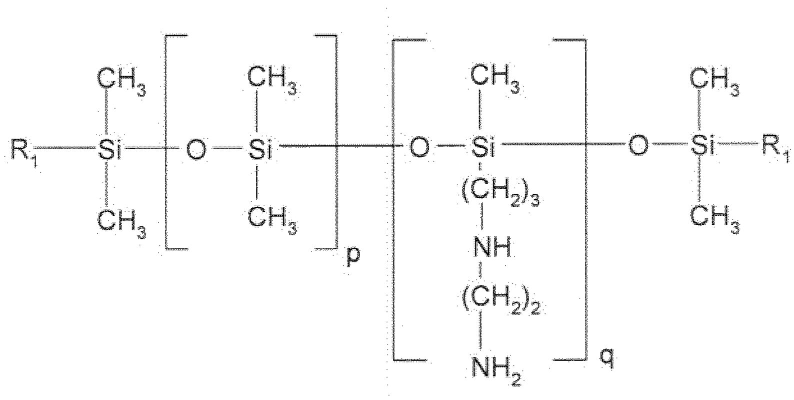

(II)

in which:

p and q are numbers such that the sum (p + q) can range especially from 1 to 1000, in particular from 50 to 350 and more particularly from 150 to 250,
p possibly denoting a number from 0 to 999, especially from 49 to 349 and more particularly from 159 to 239, and q possibly denoting a number from 1 to 1000, especially from 1 to 10 and more particularly from 1 to 5;
$R_1$ represents a hydroxyl or $C_1$-$C_4$ alkoxy radical; at least some of the radicals $R_1$ of the silicone or of the mixture of silicones of formula (II) denoting an alkoxy radical,
the amphoteric surfactant/sulfate or sulfonate anionic surfactant weight ratio ranging from 1 to 2,
the total amount of surfactants representing from 4% to 35% by weight relative to the total weight of the final composition.

2. Composition according to Claim 1, **characterized in that** the sulfate or sulfonate anionic surfactant(s) is (are) present in concentrations ranging from 2% to 15% by weight and preferably from 4% to 8% by weight relative to the total weight of the composition.

3. Composition according to either of Claims 1 and 2, **characterized in that** the amphoteric surfactant(s) is (are) present in concentrations ranging from 2% to 30% by weight and preferably from 5% to 10% by weight relative to the total weight of the composition.

4. Composition according to any one of Claims 1 to 3, **characterized in that** the amphoteric surfactant/sulfate or sulfonate anionic surfactant weight ratio ranges from 1.2 to 1.8 and more particularly from 1.3 to 1.5.

5. Composition according to any one of Claims 1 to 4, **characterized in that**, in the amino silicone of formula (I) or (II), the alkoxy radicals are methoxy radicals.

6. Composition according to any one of Claims 1 to 5, **characterized in that**, in the amino silicone or the mixture of amino silicones of formula (I), the mole ratio of the hydroxyl/alkoxy radicals of the silicone or of the mixture of silicones of formula (I) ranges from 0.2 to 0.4 and preferably from 0.25 to 0.35.

7. Composition according to any one of Claims 1 to 5, **characterized in that**, in the amino silicone or the mixture of amino silicones of formula (II), the mole ratio of the hydroxyl/alkoxy radicals of the silicone or of the mixture of silicones of formula (II) ranges from 0.1 to 0.5, preferably from 0.15 to 2 and even more particularly from 0.5 to 0.9.

8. Composition according to any one of the preceding claims, **characterized in that** the amino silicones of formula (I) or (II) are used in the form of a microemulsion.

9. Composition according to any one of the preceding claims, **characterized in that** the amino silicones of formulae (I) or (II) are present in concentrations ranging from 0.01% to 20%, preferably from 0.05% to 10% by weight and even more particularly from 0.1% to 5% by weight relative to the total weight of the composition.

10. Composition according to any one of the preceding claims, **characterized in that** it also comprises one or more carboxylic anionic surfactant(s).

11. Composition according to any one of the preceding claims, **characterized in that** the composition also comprises one or more non-silicone cationic polymers.

12. Composition according to Claim 11, **characterized in that** the cationic polymer is chosen from diallyldimethylammonium salt homopolymers and cationic polysaccharides.

13. Composition according to either of Claims 11 and 12, **characterized in that** the said cationic polymer represents from 0.005% to 10% by weight, preferably from 0.01% to 5% by weight and even more preferentially from 0.1% to 3% by weight relative to the total weight of the final composition.

14. Process for protecting the colour with respect to washing of artificially dyed keratin fibres, especially human keratin fibres and more particularly the hair, **characterized in that** it consists in applying to the said fibres, before or preferably after dyeing the said fibres, at least one composition as defined in one of Claims 1 to 13.

15. Use of a composition as defined in one of Claims 1 to 13, for protecting the colour with respect to washing of artificially dyed keratin fibres, especially human keratin fibres and more particularly the hair.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1312346 A
- US 2528378 A
- US 2781354 A
- EP 0337354 A
- FR 2270846 A
- FR 2383660 A
- FR 2598611 A
- FR 2470596 A
- FR 2519863 A